# EUROPÄISCHE PATENTSCHRIFT

(11) **EP 2 670 330 B1**
(45) Veröffentlichungstag und Bekanntmachung des Hinweises auf die Patenterteilung: **28.01.2015**
(21) Anmeldenummer: 13716281.4
(22) Anmeldetag: 12.04.2013
(51) Int. Cl.: A61B 18/14

(54) **MEDIZINISCHES HF-INSTRUMENT MIT SCHWENKBAREM ELEKTRODENLAGER**
MEDICAL HF INSTRUMENT HAVING A PIVOTABLE ELECTRODE BEARING
INSTRUMENT MÉDICAL DE HF COMPORTANT UN SUPPORT D'ÉLECTRODE PIVOTANT

(30) Priorität: 20.04.2012 DE 102012103503
(43) Veröffentlichungstag der Anmeldung: 11.12.2013
(73) Patentinhaber: Aesculap AG, 78532 Tuttlingen (DE)
(72) Erfinder: WEISSHAUPT, Dieter, 78194 Immendingen (DE); ROTHWEILER, Christoph, 78166 Donaueschingen (DE)
(74) Vertreter: Winter, Brandl, Fürniss, Hübner, Röss, Kaiser, Polte - Partnerschaft mbB
(86) Internationale Anmeldenummer: PCT/EP2013/057662
(87) Internationale Veröffentlichungsnummer: WO 2013/156400

(56) Entgegenhaltungen:
- EP-A1- 2 158 867
- WO-A2-2007/103986
- WO-A2-2010/088044
- US-A1- 2006 217 697
- US-A1- 2006 259 054
- US-A1- 2007 043 297
- US-A1- 2009 204 114

## Beschreibung

Die vorliegende Erfindung betrifft ein medizinisches HF-Instrument (TFT-Instrument) gemäß dem Oberbegriff des Anspruchs 1.

Bei der Hochfrequenz-Chirurgie, im nachfolgenden auch als HF-Chirurgie bezeichnet, wird ein Wechselstrom mit hoher Frequenz durch den menschlichen Körper bzw. eine bestimmte Gewebepartie des menschlichen Körpers geleitet, um das Gewebe an einer bestimmten Stelle gezielt zu schädigen bzw. zu schneiden. Ein wesentlicher Vorteil gegenüber einer herkömmlichen Schneidetechnik mit dem Skalpell besteht darin, dass gleichzeitig mit dem Schnitt eine Blutungsstillung durch Verschluss der betroffenen Gefäße erfolgen kann. Beispielsweise beim Resezieren von Gewebe, wie etwa bei einer Lungenteilresektion, oder bei einer Leberlappenresektion wird ein derartiger Eingriff daher nicht mehr wie üblich mittels eines Klammernahtgeräts ausgeführt, sondern das Gewebe wird mit dem HF-Strom eines derartigen TFT-Instruments "verschweißt" bzw. "versiegelt". Der Vorteil dieses Verfahrens liegt eindeutig darin, dass keine Implantate (z.B. Metallklammern) im Körper verbleiben.

Die HF-Elektrochirurgie lässt sich grundsätzlich in zwei Anwendungstechniken unterscheiden, nämlich die monopolare Anwendungstechnik sowie die bipolare Anwendungstechnik.

Am häufigsten wird derzeit noch immer die monopolare Technik angewendet. Dabei wird ein Pol der HF-Spannungsquelle über eine möglichst große Fläche mit dem Patienten verbunden. Diese Elektrode bezeichnet man als Neutralelektrode. Der andere Pol ist das eigentliche chirurgische Instrument (Aktive Elektrode), welche vom Chirurgen manuell geführt wird. Der Strom fließt über den Weg des geringsten Widerstandes von der Aktivelektrode zur Neutralelektrode. In unmittelbarer Nähe der Aktivelektrode ist die Stromdichte am höchsten, hier findet der thermische Effekt am stärksten statt.

Die neutrale Elektrode ist möglichst großflächig ausgebildet, sodass die Stromdichte im Körper gering gehalten wird und keine Verbrennungen stattfinden. Die Haut an der Neutralelektrode wird durch die große Fläche nicht spürbar erwärmt.

Bei der bipolaren Technik fließt der Strom im Gegensatz zur monopolaren Technik nur durch einen kleinen Teil des Körpers - nämlich denjenigen, in dem die chirurgische Wirkung (Schnitt oder Koagulation) gewünscht ist. Zwei gegeneinander isolierte Elektroden, zwischen denen die HF-Spannung anliegt, werden hierfür direkt an die Operationsstelle geführt. Der Stromkreis wird über das dazwischen liegende Gewebe geschlossen. In dem Gewebe zwischen den Elektroden findet dann der thermische Effekt statt.

Gegenüber der monopolaren Technik wird 20-30 % weniger Leistung benötigt. Das umgebende Gewebe wird, weil hier kein Strom fließt, nicht geschädigt und Messgeräte am Patienten (z.B. EKG) werden nicht gestört. Diese Methode ist daher für kritische und präzise Anwendungen, wie beispielsweise Mikro-, Neuro- und HNO-Chirurgie besonders gut geeignet.

Damit es insbesondere in der bipolaren Anwendungstechnik nicht zum Versagen der Versiegelung bzw. der Verschweißung des Gewebes kommt, müssen die auf das Gewebe einwirkenden Parameter erfasst und möglichst exakt am Instrument eingeregelt werden. Um dies zu gewährleisten, ist eine genaue Kontrolle der folgenden Parameter unabdingbar:
- Temperatur am/im Gewebe zwischen den Elektroden,
- Einspanndruck, der von den Elektroden auf das Gewebe ausgeübt wird,
- Gewebeimpedanz,
- Elektrodenabstand zueinander und ggf.
- Lage des Instruments bzw. der Elektroden zueinander.

Bei den Frequenzen, die für die HF-Chirurgie verwendet werden, verhält sich das Körpergewebe wie ein Ohmscher Widerstand. Der spezifische Widerstand hängt dabei stark von der Gewebeart ab, wobei sich die Leistungszufuhr bei gleicher Temperaturerhöhung des Gewebes direkt proportional zum spezifischen Widerstand verhält. Der spezifische Widerstand von Muskelgewebe und stark durchblutetem Gewebe ist relativ gering. Der von Fett ist ca. um den Faktor 15 höher und der von Knochen um den Faktor 1000. Der wirksame Widerstand hängt aber zusätzlich auch von der Elektrodenart und -form, sowie dem beabsichtigten Zerstörungsgrad des Gewebes ab. Die Form und Höhe des Stroms müssen somit auf die Gewebeart, an der operiert wird, sowie die verwendeten Elektroden genau abgestimmt werden und sollten über die gesamte Elektrodenlänge ein konstantes bzw. gleichmäßiges Ergebnis erzielen.

Die schnelle und effiziente Blutstillung bei der Koagulationsanwendung kommt bei fehlender spontaner Gerinnung zum Einsatz und ersetzt dann bei kleinen Gefäßen in den meisten Fällen den teuren Fibrinkleber oder die aufwendige Ligatur. Der Begriff Koagulation umfasst hierbei zwei verschiedene Techniken des Operierens: Die Tiefenkoagulation und die (elektrische) Blutstillung.

Bei der Tiefenkoagulation wird das Gewebe großflächig auf 50-80°C erhitzt. Das geschieht mit Kugel-, Platten- oder Walzenelektroden und dient zum späteren Abtragen des Gewebes. Man verwendet eine große Stromdichte und Strom ohne Impulsmodulation. Durch die Größe der Stromstärke kann man die Tiefe der Koagulation beeinflussen.

Bei der Blutstillung verwendet man impulsmodulierten HF-Strom an Klemmen und Pinzetten. Die Blutgefäße werden mit den Spitzen des Werkzeugs gefasst und durch die Dehydration verengt bis sie komplett verschlossen sind. Es wird im bipolaren Betrieb gearbeitet, selten werden auch monopolare Pinzetten verwendet.

Als Elektrotomie wird schließlich das Schneiden des Gewebes (statt Schneiden mit dem Skalpell) in der HF-Chirurgie bezeichnet. Beim Schneiden wird das HF-Chirurgiegerät (TFT-Instrument) mit Nadel oder schmalem Blatt (Klinge) im monopolaren Modus betrieben. Neuerdings kommen zum Schneiden auch bipolare Scheren sehr erfolgreich zur Anwendung.

Benachbarter Stand der Technik ist aus den Druckschriften DE 10 2008 008 309 A1, DE 10 2010 031 569 A1, US-A-2009/204 114, EP-A-2 158 867, WO-A-2010/088 044 und US 2008 / 0 183 251 A1 bekannt. Die DE 10 2010 031 569 A1 offenbart hierbei ein elektrochirurgisches Instrument zum Schneiden und Versiegeln von Gewebe, wobei das Instrument umfasst:
a) ein erstes und ein zweites Backenelement in gegenüberliegender Beziehung relativ zueinander, wobei das erste Backenelement eine Innenfläche aufweist, die dafür ausgelegt ist, mit der Innenfläche des zweiten Backenelements zusammenzuwirken, um dazwischen Gewebe zu greifen, wobei wenigstens eines der Backenelemente bezüglich des anderen beweglich ist, so dass die Backenelemente wahlweise zwischen einer offenen Stellung, in der die Backenelemente in einer beabstandeten Beziehung relativ zueinander angeordnet sind, und einer geschlossenen Stellung, in der die Innenflächen der Backenelemente zusammenwirken, um dazwischen Gewebe zu greifen, betätigbar sind;
b) Mittel zum Bewirken einer Bewegung des einen oder jedes Backenelements, um somit die Backenelemente zwischen der offenen und der geschlossenen Stellung zu betätigen;
c) eine erste Elektrode als Koagulationselektrode auf der Innenfläche des einen der Backenelemente;
d) eine zweite Elektrode als Koagulationselektrode auf der Innenfläche des einen der Backenelemente;
e) ein Isolationselement, das die erste und die zweite Elektrode trennt, wobei die erste und die zweite Elektrode mit entgegengesetzten Polen eines elektrochirurgischen Generators verbindbar sind;
f) eine dritte Elektrode als Schneideelektrode auf der Innenfläche des ersten Backenelements, wobei die dritte Elektrode mit einem Pol des elektrochirurgischen Generators verbindbar ist; und
g) eine vierte Elektrode auf einer Aussenfläche des ersten Backenelements getrennt von der Innenfläche, wobei die vierte Elektrode mit einem Pol des elektro-chirurgischen Generators verbindbar ist;
wobei das elektrochirurgische Instrument fähig ist, wahlweise eine Koagulation des Gewebes zwischen der ersten und der zweiten Elektrode und/oder ein Schneiden des von der dritten Elektrode berührten Gewebes und/oder ein Behandeln des von der vierten Elektrode berührten Gewebes zu bewirken.

Bei den chirurgischen HF-Instrumenten (TFT-Instrumenten) der bipolaren Bauart vorzugsweise bestehend aus zwei scheren- oder zangenförmig schwenkbaren Elektrodenbranchen besteht jedoch generell das Problem, dass es beim Schließen des aus den Elektrodenbranchen aufgebauten Maulteils zu einer nicht exakt parallelen Ausrichtung der sich gegenüberliegenden HF-Elektroden (Versiegelungs-/Verschweißungselektroden) kommt, sodass der auf das dazwischen eingeklemmte Gewebe ausgeübte Einklemmdruck längs der Elektroden nicht homogen ist. Da der Einklemmdruck einer jener vorstehend genannten Parameter ist, der das Behandlungsergebnis nachweislich sehr beeinflusst, hat ein inhomogener Einklemmdruck eine negative Auswirkung auf die Nahtqualität längs der beiden Elektroden. Außerdem bedeutet eine ungleichmäßige Spaltweite einen ungleichmäßigen Stromfluss über die Elektrodenlänge, was ebenfalls nachteilig auf das Behandlungsergebnis (Koagulationsqualität) ist. Dabei sind die Einflüsse der genannten Parameter auf das Behandlungsergebnis derart intensiv, dass bereits geringe Abweichungen von den Optimalwerten erhebliche Auswirkungen zeigen können.

Angesichts dieser Problematik ist es eine Aufgabe der vorliegenden Erfindung, ein chirurgisches, bipolares HF-Instrument (TFT-Instrument) vorzugsweise der Scheren- oder Zangenbauart bereitzustellen, bei welchem die sich gegenüberliegenden, sowie zusammenwirkenden HF-Elektroden beim Schließen des Maulteils parallel zueinander liegen, wodurch über die wirksame Elektrodenlänge gleichbleibende Voraussetzungen für die HF-Chirurgie (Gewebeimpedanz) geschaffen werden sollen. Ein Ziel ist es hierbei, die Qualität der Gewebeversiegelungen/ -verschweißungen in genauerer Weise elektrisch kontrollieren zu können.

Diese Aufgabe wird durch ein medizinisches HF-Instrument mit den Merkmalen des Anspruchs 1 gelöst. Vorteilhafte Ausgestaltungen der Erfindung sind Gegenstand der Unteransprüche.

Gemäß einem Aspekt der vorliegenden Erfindung wird zur Lösung der vorstehenden Aufgabe ein medizinisches HF-Instrument der bipolaren Bauart vorgeschlagen mit zwei aufeinander zu bewegbaren Elektrodenbranchen oder Elektroden-(Basis)trägern, an denen jeweils zumindest eine längs sich erstreckende Elektrode oder Elektrodenreihe (Koagulationselektroden / -elektrodenreihen) angeordnet ist, die zueinander hinweisen. Zumindest eine Elektrode oder Elektrodenreihe ist an einer separaten sowie dreh-, schwenk- oder kippbar an einer der Elektrodenbranchen/ -basisträgern gelagerten ( oder schwimmend gelagerten) Elektrodenhalterung montiert, die sich demnach vorzugsweise selbsttätig parallel zur gegenüberliegenden Elektrode ausrichten kann oder die in Abstimmung auf die Bewegung der Elektrodenbranchen/ -basisträger sowie auf die beabsichtigte Spaltweite zwischen den Elektroden im geschlossenen Zustand des Instruments so eingestellt ist, dass eine parallele Ausrichtung der Elektroden im geschlossenen Zustand des Instruments erreicht wird. Erfindungsgemäß ist zumindest diese eine, an der separaten Elektrodenhalterung montierte Elektrode oder Elektrodenreihe (über deren Elektrodenlänge hinweg) federnd/nachgiebig an der Elektrodenhalterung gelagert. Durch diesen eingebauten Federmechanismus zwischen der zumindest einen Elektrode und der separaten Elektrodenhalterung wird die Parallelität der Elektroden im geschlossenen Zustand des Instruments weiter verbessert, sodass der Abstand der Elektroden über deren gesamte wirksame Länge vergleichmäßigt ist. Durch den so eingestellten/einstellbaren (über die Elektrodenlänge im Wesentlichen konstanten) Elektrodenabstand kommt es zwangsläufig zu einer gleichmäßigen Durchdringung des (eingespannten) Gewebes mit HF-Energie.

Die dadurch erzielbaren Vorteile lassen sich wie Folgt zusammenfassen:
- Zunächst werden nicht beabsichtigte (übergebührliche oder zu geringe) Gewebeschäden verhindert, die durch eine überhöhte/verminderte Krafteinwirkung auf das eingespannte Gewebe erzeugt werden könnten. D.h., der von den Elektroden ausgeübte Einspanndruck lässt sich grundsätzlich besser kontrollieren.
- Es wird eine sichere Fusion der einzelnen Gewebebestandteile erreicht, insbesondere durch gleichbleibende sowie reproduzierbare Kraftverhältnisse.
- Die Qualität der Gewebefusion verbessert sich auch dadurch, dass der Elektrodenabstand über die wirksame Elektrodenlänge gleich ist, sodass ein im Wesentliche exakt vorbestimmter/verbessert vorbestimmbarer HF-Strom zwischen den Elektroden an jeder Elektroden-Längsposition fließen kann.

Ein optional zusätzlicher oder unabhängiger Aspekt der Erfindung zur Lösung der vorstehenden Aufgabe sieht vor, die separate Elektrodenhalterung vorzugsweise im Fall eines zangen- oder scherenförmigen TFT-Instruments mit leistenförmigen (d.h. axial längs sich erstreckenden, geraden) Elektroden an ihrem distalen Endabschnitt / Ende dreh-, schwenk- oder kippbar an der jeweiligen Elektrodenbranche zu lagern, sodass die Elektrodenhalterung auch entgegen der Schwenkbewegung der betreffenden Elektrodenbranche an dieser relativ drehen kann. D.h. beim Betätigen des HF-Instruments werden die Elektrodebranchen an ihren proximalen Endabschnitten zueinander verschwenkt. Dem gegenüber ist die separate Elektrodenhalterung vorzugsweise an ihrem distalen Endabschnitt an der betreffenden Branche angelenkt und ist somit auch entgegen der Schließ-Schwenkbewegung der Branche drehbar. Vorzugsweise ist bei dieser besonderen Ausführungsform am proximalen Endabschnitt der separaten Elektrodenhalterung ein (verstellbarer) Stützmechanismus zwischen der Elektrodenhalterung und der Branche angeordnet, sodass der wirksame (spitze) Winkel zwischen der Elektrodenhalterung und der Branche einregelbar ist.

An dieser Stelle sei natürlich darauf hingewiesen, dass der Schwenk-, Dreh- oder Kipppunkt der Elektrodenhalterung an der Branche nicht notwendiger Weise am distalen Endabschnitt der Elektrodenhalterung angeordnet sein muss, sondern sich auch in deren Mitten- oder proximalem Endabschnitt befinden kann.

Gemäß einer bevorzugten Weiterbildung der Erfindung sind zwischen der Elektrodenhalterung und der daran montierten Elektrode/Elektrodenreihe eine Anzahl von Federelementen, beispielsweise Spiralfedern, Blattfedern oder Elastomerelemente eingesetzt, welche die Elektrode/Elektrodenreihe von der Elektrodenhalterung in Richtung hin zur gegenüberliegenden Elektrode/Elektrodenreihe beabstanden. Die Federelemente können dabei jeweils paarweise zusammengefasst sein, um eine größere gemeinsame Aufstandsfläche zusimulieren, wobei die Federelementpaare gleichmäßig in Elektrodenlängsrichtung voneinander beabstandet sind.

Weiter vorzugsweise sind die Federelemente, insbesondere in Spiralfederausführung in Aufnahmetaschen eingesetzt, welche in der Elektrodenhalterung ausgebildet sind. Zumindest die an der separaten Elektrodenhalterung montierte Elektrode hat in ihrer Draufsicht eine U-Form, wobei sich zwischen den parallel zueinander in Branchenlängsrichtung sich erstreckenden Elektrodenschenkeln ein Spalt in Breitenrichtung der Elektrodenhalterung ergibt, in welchem eine weitere, blatt- oder klingenförmige Elektrode in isolierter Weise gelagert/geführt ist, die als Schneidelektrode dient. Demzufolge hat die Elektrodenbranche sowie die daran gelagerte separate Elektrodenhalterung ebenfalls einen Längsschlitz im Wesentlichen deckungsgleich zum Elektrodenspalt, durch den die weitere Schneidelektrode isolierend geführt ist, so dass diese unabhängig von der Elektrodenbranche/Elektrodenhalterung für einen Schneideingriff mit einem eingespannten Gewebe bewegbar ist.

Schließlich kann gemäß einem zusätzlichen oder alternativen Aspekt der Erfindung eine Abstands-Einstelleinrichtung an zumindest einer der Branchen, vorzugsweise an der Branche mit der separaten Elektrodenhalterung, vorgesehen sein, mittels der ein Elektrodenabstand bei geschlossenem Maulteil des HF-Instruments einstellbar / veränderbar ist. Vorzugsweise betrifft diese Abstands-Einstelleinrichtung eine Stellschraube, die außerhalb der wirksamen Länge der Elektroden in die eine Branche bevorzugt an deren distalem Ende eingedreht ist und die sich an der gegenüberliegenden Branche abstützt. Alternativ hierzu ist es aber auch möglich, an dem die Branchen schwenkbar koppelnden Scharnier einen verstellbaren Anschlag vorzusehen, mit welchem der maximale Schwenkwinkel in Schließrichtung der Branchen einstellbar ist.

An dieser Stelle sei noch darauf hingewiesen, dass die Federelemente aus einem thermisch leitfähigen Material bestehen können und somit vorzugsweise als Wärmeübertragungselemente dienen. Dadurch kann Wärme von den Elektroden über die Federelemente in die Elektrodenhalterung abgeleitet werden. Die ableitbare Wärmemenge kann ferner über die Zahl der eingebauten Federelemente eingestellt werden.

Die Erfindung wird nachstehend anhand bevorzugter Ausführungsbeispiele unter Bezugnahme auf die begleitenden Figuren näher erläutert.
Fig. 1 zeigt ein erstes bevorzugtes Ausführungsbeispiel eines HF-Instruments gemäß der Erfindung in teilweise aufgebrochener Seitenansicht ohne eine Elektrodenabstands-Einstelleinrichtung,
Fig. 2 zeigt ein zweites bevorzugtes Ausführungsbeispiel eines HF-Instruments gemäß der Erfindung in Seitenansicht mit einer Elektrodenabstands-Einstelleinrichtung,
Fig. 3 zeigt ein Maulteil des HF-Instruments gemäß der Fig. 1 in vergrößerter, teilweise aufgebrochener Seitenansicht,
Fig. 4 zeigt den distalen Endabschnitt des Maulteils gemäß der Fig. 3 in weiterer Vergrößerung,
Fig. 5 zeigt ein Maulteil des HF-Instruments gemäß der Fig. 2 in vergrößerter, teilweise aufgebrochener Seitenansicht,
Fig. 6. zeigt das Maulteil gemäß Fig. 5 in Seitenansicht mit Funktionsdarstellung der Elektrodenabstands-Einstelleinrichtung und
Fig. 7 zeigt eine Branche mit einer daran anscharnierten Elektrodenhalterung und Elektroden in einer Draufsicht.

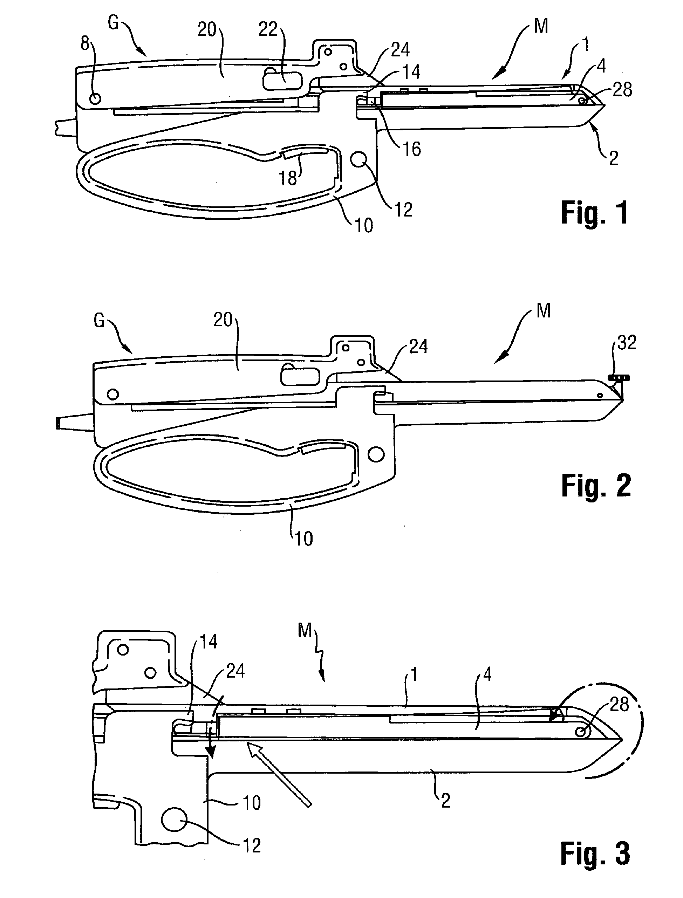

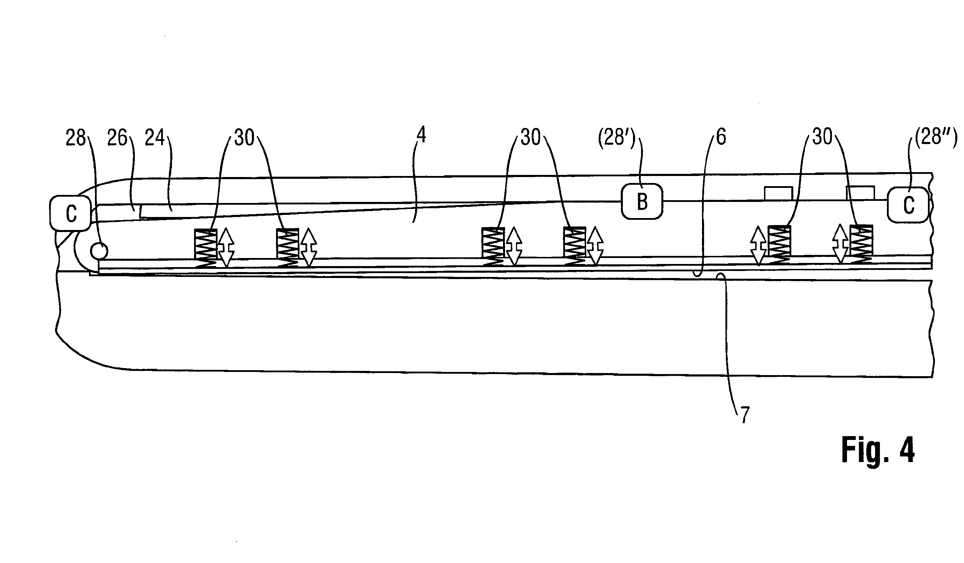

Das in der Fig. 1 beispielhaft dargestellte HF-Instrument (der Bipolarbauart) gemäß einer ersten bevorzugten Ausführungsform der Erfindung hat prinzipiell ein Maulteil M bestehend aus zwei pinzetten-, zangen- oder scherenförmig zueinander bewegbaren (langgestreckten) Elektrodenbranchen 1, 2 und einen Instrumentengriff G zur Betätigung (und elektrischen Aktivierung) des Maulteils M. An (zumindest) einer Elektrodenbranche 1 ist eine separate Elektrodenhalterung 4 (relativ hierzu verschwenkbar) anscharniert, an der wiederum eine erste Elektrode 6 (siehe Fig. 4) federnd/nachgiebig montiert ist.

Im Konkreten besteht das HF-Instrument gemäß der Fig. 1 aus einer ersten (gemäß der Fig. 1 oberen) Elektrodenbranche 1 und einer zweiten (gemäß der Fig. 1 unteren) Elektrodenbranche 2, die an ihren proximalen Enden (entspricht auch dem proximalen Ende des HF-Instruments insgesamt) scharnierartig mittels eines Scharnierbolzens 8 aneinandergelenkt sind. Im distalen Abschnitt des HF-Instruments bilden die beiden

Branchen 1, 2 das Maulteil M und im proximalen Abschnitt des HF-Instruments bilden die Branchen 1, 2 den Instrumentengriff G.

Im Bereich des Instrumentengriffs G ist an der zweiten (unteren) Branche 2 in deren Mittenabschnitt eine erste bügelartige Handhabe (Handhebel) 10 über einen Bolzen 12 anscharniert, an der ein haken- oder dornförmiger Riegel 14 angeformt ist, der mit Hinterschneidungen oder Zapfen/Vorsprüngen 16 im Mittenbereich der ersten (oberen) Branche 1 in Rasteingriff bringbar ist, um diese in einer geschlossenen Position mit der zweiten Branche 2 zu verriegeln. In der bügelartigen Handhabe 10 ist eine Betätigungstaste 18 relativ bewegbar integriert, über die ein (nicht weiter gezeigter) elektrischer Schalter zur Beaufschlagung von Elektroden/Elektrodenreihen an den Branchen 1, 2 mit HF-Strom (zum Verschweißen/Versiegeln) vorzugsweise individuell aktivierbar ist. Schließlich ist im Bereich des Instrumentengriffs G an der ersten (oberen) Branche 1 bzw. am proximalen Scharnierbolzen 8 eine zweite Handhabe (Handhebel) 20 anscharniert, die relativ zu den beiden Branchen 1, 2 betätigbar/schwenkbar ist. In der zweiten Handhabe 20 ist ein Sperrelement oder Sperrbolzen 22 integriert, der relativ zur zweiten Handhabe 20 bewegbar ist, um von einer Verriegelungsposition, in welcher der Sperrbolzen 22 die zweite Handhabe 20 mit der ersten (oberen) Branche 1 verriegelt, in eine Entriegelungsposition geschaltet werden zu können, in welcher die zweite Handhabe 20 relativ zu den Branchen 1, 2 bewegbar / verschwenkbar ist. An der zweiten Handhabe 20 ist eine klingenförmige Schneidelektrode 24 gekoppelt (fixiert), die durch die zweite Handhabe 20 unabhängig zu den Branchen 1, 2, bewegbar ist. Vorzugsweise ist an der zweiten Handhabe 20 ebenfalls eine nicht weiter dargestellte Betätigungstaste angeordnet, über die ein weiterer elektrischer Schalter (nicht gezeigt) zum Beaufschlagen der Schneidelektrode 24 mit HF-Strom (individuell) aktivierbar ist.

Die Fig. 3 und 4 zeigen den konstruktiven Aufbau des Maulteils M gemäß der Fig. 1 im Detail.

Demnach ist an der ersten (oberen) Branche 1 die separate Elektrodenhalterung 4 vorzugsweise in Form einer Halteleiste über ein Scharnier 28 angelenkt, das am distalen Endabschnitt der ersten Branche 1 angeordnet ist. Hierfür ist die erste Branche 1 mit einer Längsnut 26 ausgebildet, in welcher die Halteleiste 4 schwenkbar um das Scharnier 28 aufgenommen ist. Des Weiteren ist an der ersten Branche 1 ein nicht im Einzelnen dargestellter Verstellmechanismus vorgesehen, mittels welchem die Relativlage (bzw. der Relativwinkel) der Elektrodenhalterung/Halteleiste 4 zur ersten Branche 1 justierbar ist.

An beiden Branchen 1, 2 ist gemäß der Fig. 4 im Bereich des Maulteils M jeweils eine zungenförmige Elektrode oder Elektrodenreihe 6, 7 angeordnet, die sich gegenüberliegen sowie parallel zueinander ausgerichtet sind. Im Konkreten ist die erste (obere) Elektrode 6 an der Elektrodenhalterung 4 vorzugsweise aus elektrisch nichtleitendem Material in elektrisch isolierter Weise montiert, wohingegen die zweite (untere) Elektrode 7 vorliegend unmittelbar an der zweiten Branche 2 in elektrisch isolierter Weise montiert ist. Demnach ist die erste Elektrode 6 zusammen mit der Elektrodenhalterung 4 relativ zur ersten Branche 1 verschwenkbar, um so bezüglich der zweiten Elektrode 7 ausgerichtet werden zu können, wie dies nachfolgend noch im Einzelnen näher beschrieben wird.

Die Lagerung der ersten Elektrode 6 an der zugehörigen separaten Elektrodenhalterung 4 erfolgt im vorliegenden Beispiel federelastisch mittels einer Anzahl von Federelementen 30, die vorliegend paarweise (oder auch einzeln) in Elektrodenlängsrichtung beabstandet sind. Jedes Federelement 30 ist in einer Aufnahmetasche/Sackloch aufgenommen bzw. gelagert, die in der Elektrodenhalterung 4 ausgeformt sind. Die Federelemente 30 üben eine Vorspannkraft auf die Flachseite der ersten Elektrode 6 aus, um diese in Richtung hin zur zweiten Elektrode bezüglich der Elektrodenhalterung 4 elastisch abzustützen.

An dieser Stelle sei aber darauf hingewiesen, dass an Stelle der Federelastizität der Lagerung auch eine ausschließlich flexible Lagerung (beispielsweise durch Befüllen des Raums zwischen der Elektrodenhalterung und der Elektrode mit einem Dämmstoff) möglich ist, wobei die Rückstellkraft vorzugsweise aus der Eigenelastizität der Elektrode herrührt. Im gezeigten Ausführungsbeispiel sind die Federelemente 30 des Weiteren als Spiralfedern dargestellt. Es können aber auch Blattfedern oder Elastomere verwendet werden. Grundsätzlich wäre es aber auch denkbar zwischen der ersten Elektrode 6 und der Elektrodenhalterung 4 eine nachgiebige, vorzugsweise federnde Trennschicht, beispielsweise eine elastische Matte zwischen zu fügen.

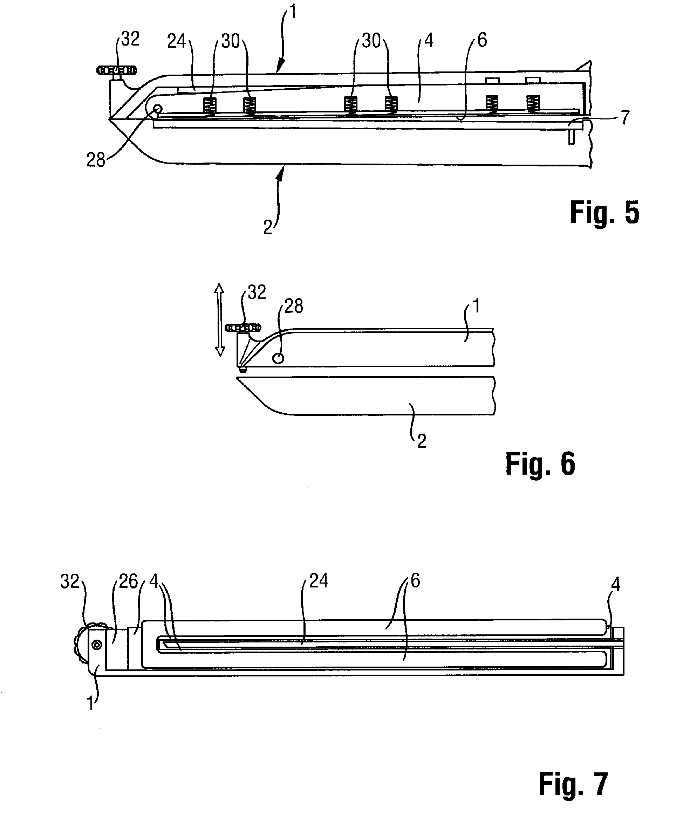

In der Fig. 7 ist der generelle Grundriss der Elektroden an der ersten (oberen) Branche 1 dargestellt, wie er für alle gezeigte Ausführungsbeispiele der Erfindung gilt.

Demnach ist die flexibel an der Elektrodenhalterung 4 gelagerte Elektrode/Elektrodenreihe 6 in der Draufsicht zu einer U-Form gebildet, deren Schenkel sich unter Ausformung eines (mittigen) Längsspalts in Branchenlängsrichtung erstrecken. Die erste Branche 1 ist ebenfalls mit einem durchgehenden Längsschlitz im Wesentlichen deckungsgleich zum Längsspalt der ersten Elektrode 6 ausgeformt, in den die Schneidelektrode 24 relativ bewegbar sowie in isolierter Weise eingeführt ist.

Die Funktionsweise des HF-Instruments gemäß dem ersten bevorzugten Ausführungsbeispiel der vorliegenden Erfindung lässt sich wie Folgt umschreiben:

Zunächst wird die Elektrodenhalterung 4 an dem nicht weiter gezeigten Einstellmechanismus bezüglich der ersten (oberen) Branche 1 so justiert (am Scharnier 28 relativ verschwenkt), dass die erste Elektrode 6 im geschlossenen Zustand des HF-Instruments eine bestimmte Spaltweite zur zweiten Elektrode 7 ausbildet, die vorzugsweise über die gesamte wirksame Elektrodenlänge im Wesentlichen konstant ist.

Um nunmehr ein zu behandelndes Gewebe im Maulteil M zu fixieren, werden die Gewebeteile zwischen die Elektroden 6, 7 längs der Branchen 1, 2 eingeführt, worauf anschließend die erste Handhabe 10 betätigt wird, um die erste und zweite Branchen 1, 2 über den Riegel 14 zu verspannen/verrasten. In dieser Position gemäß der Fig. 1 hintergreift der Riegel 14 den Vorsprung 16 an der ersten (oberen) Branche 1 und spannt diese in deren Mittenabschnitt gegen die zweite (untere) Branche 2 vor. Da die beiden Branchen 1, 2 am proximale Ende über den Bolzen 8 aneinander scharniert sind und an ihrem distalen Ende einen vordefinierten Aufstandpunkt (nicht dargestellt) aufweisen, ergibt sich zwischen den Branchen 1, 2 ein vordefinierter Spalt, dessen Spaltweite an der Elektrodenhalterung 4 bereits eingestellt ist. In dieser verriegelten Position wird das zu verschweißende/versiegelnde Gewebe zwischen den Branchen 1, 2 eingespannt, wobei die beiden Elektroden / Elektrodenreihen 6, 7 eine bestimmte Einspannkraft (Einspanndruck) auf das Gewebe ausüben.

Mit Betätigen der Taste 18, werden die beiden genannten Elektroden 6, 7 mit HF-Strom beaufschlagt, der in Abhängigkeit der eingangs bezeichneten Randbedingungen durch das eingeklemmte Gewebe strömt und dieses vordefiniert schädigt. Hierdurch wird beispielsweise ein Verschweißen/Versiegeln des Gewebes erreicht,

Nach Freigeben der Taste 18 wird der Sperrbolzen 22 entriegelt und damit die zweite Handhabe 20 freigegeben. Diese kann nunmehr relativ zu der ersten Branche 1 in Richtung hin zur zweiten Branche 2 verschwenkt werden, wobei die daran fixierte blatt-oder zungenförmige Schneidelektrode 24 in den Längsspalt zwischen den Schenkeln der ersten Elektrode 4 eintaucht und gegen das eingeklemmte Gewebe drückt. Wird nunmehr diese Schneidelektrode 24 mit HF-Strom beaufschlagt, wird das Gewebe zwischen den Branchen 1, 2 durchtrennt.

Auf diese Weise kann das eingeklemmte Gewebe verschweißt/versiegelt und/oder geschnitten werden, beispielsweise um überstehende Gewebelappen/-ränder abzutrennen.

Im Nachfolgenden wird anhand der Fig. 2, 5, 6 und 7 ein zweites bevorzugtes Ausführungsbeispiel der Erfindung beschrieben, wobei im Wesentlichen nur auf die konstruktiven und funktionellen Unterschiede zum ersten Ausführungsbeispiel eingegangen werden soll. Alle weiteren technischen Merkmale entsprechen dem zuvor beschriebenen ersten bevorzugten Ausführungsbeispiel.

Demnach ist an einer Branche, vorzugsweise der ersten (oberen) Branche 1 an deren distalem Ende sowie außerhalb der ersten Elektrode 6 / des Elektrodenhalters 4 eine Branchenabstands-Einstelleinrichtung 32 vorliegend in Form einer Stellschraube vorgesehen, die unmittelbar in die erste Branche 1 eingedreht ist und in Richtung hin zur zweiten Branche 2 vorragt, Die Stellschraube 32 ersetzt/definiert somit den zuvor beschriebenen distalen Aufstandspunkt zwischen den beiden Branchen 1, 2. In diesem Fall jedoch ist der Aufstandspunkt nicht fix sondern flexibel/einstellbar gestaltet, sodass der Branchenabstand im Bereich des Maulteils M bei geschlossenem Instrument justiert und damit auf das zu behandelnde Gewebe abgestimmt/angepasst werden kann.

Die Funktionsweise des HF-Instruments gemäß dem zweiten Ausführungsbeispiel ist weitestgehend die Gleiche wie die des ersten Ausführungsbeispiels, sodass an dieser Stelle auf die vorstehenden Textstellen verwiesen werden kann. Als zusätzlich Funktion sie jedoch die Branchenabstands-Einstelleinrichtung 32 genannt, die als ein erster Schritt betätigt wird um einen bestimmten Branchenabstand, angepasst an das zu behandelnde Gewebe einzustellen. Entsprechend dieser Voreinstellung wird dann die Einstelleinrichtung zur Verschwenkung der Elektrodenhalterung 4 betätigt, um eine Parallelität der Elektroden 6, 7 im geschlossenen Zustand der HF-Instruments zu erreichen.

Abschließend sei darauf hingewiesen, dass einige technische Merkmale gemäß vorstehender Beschreibung auch abgewandelt werden können, wie dies nachfolgend beschrieben ist.

Das Scharnier 28 zwischen der Elektrodenhalterung 4 und der ersten Branche 1 ist in beiden Ausführungsbeispielen an einer distalen Endposition der Elektrodenhalterung dargestellt, Alternativ kann das Scharnier 28' aber auch in einem Mittenabschnitt angeordnet sein, oder das Scharnier 28" befindet sich in einem proximalen Endabschnitt der Elektrodenhalterung 4 nahe dem Instrumentengriff G.

Das HF-Instrument ist als Handinstrument gezeigt, bei welchem die Branchen 1, 2 gleichzeitig den Instrumentengriff G ausbilden. Prinzipiell ist es aber auch möglich das Grundprinzip der Erfindung gemäß der vorstehenden Beschreibungseinleitung bei einem minimal-invasiven Instrument anzuwenden, bei dem das Maulteil M über einen zwischengefügten Instrumentenschaft mit dem Instrumentengriff G gekoppelt ist.

Schließlich ist es auch möglich, beide Branchen 1, 2 mit separaten Elektrodenträgern auszurüsten und diese entsprechend zu justieren.

Zusammenfassend wird erfindungsgemäß ein vorzugsweise manuell betätigbares HF-Instrument der Bipolarbauart vorgeschlagen mit einem Maulteil bestehend aus zumindest zwei pinzetten-, zangen- oder scherenförmig zueinander bewegbaren Elektrodenbranchen und einem Instrumentengriff zur mechanischen Betätigung und elektrischen Aktivierung des Maulteils bzw. der Maulteilelektroden. An zumindest einer Elektrodenbranche des Maulteils ist eine separate Elektrodenhalterung relativ zur Branche verschwenkbar anscharniert, an der wiederum eine Elektrode oder Elektrodenreihe bestehend aus mehreren seriell angeordneten Einzelelektroden federnd/nachgiebig montiert ist.

## Patentansprüche

1. Medizinisches HF-Instrument der Bipolarbauart mit zwei aufeinander zu bewegbaren Elektrodenträgern (1, 2), an denen jeweils zumindest eine längs sich erstreckende Versiegelungs- oder Verschweißungselektrode oder -elektrodenreihe (6, 7) angeordnet ist, wobei das HF-Instrument eine separate Elektrodenhalterung aufweist, die dreh-, schwenk- oder kippbar an einem der Elektrodenträger (1, 2) gelagert ist, **dadurch gekennzeichnet, dass** zumindest eine Versiegelungs- oder Verschweißungselektrode oder -elektrodenreihe (6) nachgebend an der separaten Elektrodenhalterung (4) gelagert ist.

2. Medizinisches HF-Instrument nach Anspruch 1, **dadurch gekennzeichnet, dass** die zumindest eine Elektrode oder Elektrodenreihe (6) federelastisch an der Elektrodenhalterung (4) gelagert ist.

3. Medizinisches HF-Instrument nach Anspruch 1 oder 2, **dadurch gekennzeichnet, dass** die Elektrodenträger (1, 2) als sich in Instrumentenlängsrichtung erstreckende Elektrodenbranchen ausgebildet sind, die mit einem Instrumentengriff für eine pinzetten-, zangen- oder scherenförmige Bewegung gekoppelt oder ausgeformt sind.

4. Medizinisches HF-Instrument nach Anspruch 3, **dadurch gekennzeichnet, dass** die separate Elektrodenhalterung (4) mit einer sich in Instrumentenlängsrichtung erstreckenden, geraden Elektrode oder Elektrodenreihe (6) versehen ist.

5. Medizinisches HF-Instrument nach Anspruch 4, **dadurch gekennzeichnet, dass** die Elektrodenhalterung (4) am distalen Endabschnitt der betreffenden Elektrodenbranche (1) schwenkbar anscharniert ist.

6. Medizinisches HF-Instrument nach Anspruch 5, **dadurch gekennzeichnet, dass** am proximalen Endabschnitt der separaten Elektrodenhalterung (4) ein vorzugsweise verstellbarer Stützmechanismus zwischen der Elektrodenhalterung (4) und der betreffenden Elektrodenbranche (1) angeordnet ist, sodass der wirksame Schwenkwinkel zwischen der Elektrodenhalterung (4) und der Elektrodenbranche (1) einregelbar ist.

7. Medizinisches HF-Instrument nach einem der vorstehenden Ansprüche, **dadurch gekennzeichnet, dass** zwischen der Elektrodenhalterung (4) und der daran montierten Elektrode/Elektrodenreihe (6) eine Anzahl von Federelementen (30) vorzugsweise Spiralfedern, Blattfedern oder Elastomerelemente eingesetzt sind, welche die Elektrode/Elektrodenreihe (6) von der Elektrodenhalterung (4) in Richtung hin zur gegenüberliegenden Elektrode/Elektrodenreihe (7) beabstanden.

8. Medizinisches HF-Instrument nach Anspruch 7, **dadurch gekennzeichnet, dass** die Federelemente (30) jeweils paarweise zusammengefasst sind, wobei die Federelementpaare gleichmäßig in Elektrodenlängsrichtung voneinander beabstandet sind.

9. Medizinisches HF-Instrument nach Anspruch 7 oder 8, **dadurch gekennzeichnet, dass** die Federelemente (30), insbesondere in Spiralfederausführung in Aufnahmetaschen eingesetzt sind, welche in der Elektrodenhalterung (4) ausgebildet sind.

10. Medizinisches HF-Instrument nach einem der vorstehenden Ansprüche, **dadurch gekennzeichnet, dass** die an der separaten Elektrodenhalterung (4) montierte Elektrode oder Elektrodenreihe (6) in ihrer Draufsicht eine U-Form hat, wobei sich zwischen den sich parallel zueinander erstreckenden Elektrodenschenkeln ein Spalt in Breitenrichtung der Elektrodenhalterung (4) ergibt.

11. Medizinisches HF-Instrument nach Anspruch 10, **dadurch gekennzeichnet, dass** der Elektrodenträger (1) sowie die daran montierte separate Elektrodenhalterung (4) jeweils einen Längsschlitz im Wesentlichen deckungsgleich zum Elektrodenspalt hat, durch den eine vorzugsweise klingenförmige Schneidelektrode (24) isolierend geführt ist, so dass diese unabhängig von dem Elektrodenträger (1) und der daran montierten separaten Elektrodenhalterung (4) für einen Schneideingriff mit einem zwischen den Elektrodenträgern (1, 2) eingespannten Gewebe bewegbar ist.

12. Medizinisches HF-Instrument nach einem der vorstehenden Ansprüche, **dadurch gekennzeichnet, dass** eine manuell betätigbare Abstands-Einstelleinrichtung (32) an zumindest einem der Elektrodenträger (1, 2), vorzugsweise an einem branchenförmigen Elektrodenträger (1) bestückt mit der separaten Elektrodenhalterung (4) vorgesehen ist, mittels der ein minimaler Elektrodenabstand einstellbar und/oder veränderbar ist.

13. Medizinisches HF-Instrument nach einem der Ansprüche 7 bis 9, **dadurch gekennzeichnet, dass** die Federelemente (30) gleichzeitig als Wärmeleitelemente ausgebildet sind, die mit der dadurch abgestützten Elektrode oder Elektrodenreihe (6, 7) und der Elektrodenhaltung thermisch gekoppelt sind.

## Claims

1. A medical HF instrument in bipolar construction comprising two electrode carriers (1, 2) which can be moved toward each other and are each provided with at least one longitudinally extending sealing-/welding electrode or electrode array (6, 7), wherein the HF instrument has a separate electrode mount supported on one of the electrode carriers (1, 2) in rotatable, pivotable or tilting fashion, **characterized in that** at least one sealing-/welding electrode or electrode array (6) is pliably supported on the separate electrode mount (4).

2. The medical HF instrument according to claim 1, **characterized in that** the at least one electrode or electrode array (6) is supported on the electrode mount (4) in spring-elastic manner.

3. The medical HF instrument according to claim 1 or 2, **characterized in that** the electrode carriers (1, 2) are formed as electrode legs which extend in the longitudinal direction of the instrument and are coupled to or shaped with an instrument handle for a forceps-, pincers- or scissor-like motion.

4. The medical HF instrument according to claim 3, **characterized in that** the separate electrode mount (4) is provided with a straight electrode or electrode array (6) extending in the longitudinal direction of the instrument.

5. The medical HF instrument according to claim 4, **characterized in that** the electrode mount (4) is pivotally articulated on the distal end portion of the respective electrode leg (1).

6. The medical HF instrument according to claim 5, **characterized in that**, at the proximal end portion of the separate electrode mount (4), a preferably adjustable supporting mechanism is arranged between the electrode mount (4) and the respective electrode leg (1), so that the effective pivoting angle between the electrode mount (4) and the electrode leg (1) can be adjusted.

7. The medical HF instrument according to any of the preceding claims, **characterized in that** a number of spring elements (30), preferably spiral springs, leaf springs or elastomer elements, are inserted between the electrode mount (4) and the electrode/electrode array (6) installed thereon, said spring elements keeping the electrode/electrode array (6) spaced from the electrode mount (4) toward the opposing electrode/electrode array (7).

8. The medical HF instrument according to claim 7, **characterized in that** the spring elements (30) are grouped in pairs in each case, the spring element pairs being uniformly spaced from each other in the longitudinal direction of the electrodes.

9. The medical HF instrument according to claim 7 or 8, **characterized in that** the spring elements (30), especially when realized in a spiral spring design, are inserted in accommodation pockets formed in the electrode mount (4).

10. The medical HF instrument according to any of the preceding claims, **characterized in that** the electrode or electrode array (6) installed on the separate electrode mount (4) has a U-shape when seen in top view, a gap in the width direction of the electrode mount (4) being created between the electrode legs extending parallel to each other.

11. The medical HF instrument according to claim 10, **characterized in that** the electrode carrier (1) as well as the separate electrode mount (4) installed thereon each comprise a longitudinal slit which is substantially congruent with the electrode gap, a preferably blade-shaped cutting electrode (24) being inserted in said slit in insulating manner, so that said cutting electrode can be moved independently of the electrode carrier (1) and the separate electrode mount (4) installed thereon, for a cutting engagement with any tissue clamped between the electrode carriers (1, 2).

12. The medical HF instrument according to any of the preceding claims, **characterized in that** a manually operable distance adjustment device (32) is provided on at least one of the electrode carriers (1, 2), preferably on a leg-shaped electrode carrier (1) equipped with the separate electrode mount (4), by means of which a minimum electrode spacing can be adjusted and/or altered.

13. The medical HF instrument according to any of the claims 7 to 9, **characterized in that** the spring elements (30) are simultaneously formed to be heat conducting elements which are thermally coupled to the electrode or electrode array (6, 7), supported thereby, and to the electrode mount.

## Revendications

1. Instrument médical HF de type bipolaire, comportant deux porte-électrode (1, 2) mobiles l'un vers l'autre, sur chacun desquels est disposé respectivement au moins une électrode de scellement ou soudure ou un ensemble d'électrodes de scellement ou soudure (6, 7), s'étendant dans le sens longitudinal, ledit instrument HF comportant un support d'électrodes séparé, qui est monté sur l'un des porte-électrode (1, 2) de manière à pouvoir tourner, pivoter ou basculer, **caractérisé en ce qu'**au moins une électrode de scellement ou soudure ou un ensemble d'électrodes de scellement ou soudure (6) sont montés élastiquement sur le support d'électrodes (4) séparé.

2. Instrument médical HF selon la revendication 1, **caractérisé en ce que** ladite au moins une électrode ou l'ensemble d'électrodes (6) sont montés de manière élastiquement flexible sur le support d'électrodes (4).

3. Instrument médical HF selon la revendication 1 ou 2, **caractérisé en ce que** les porte-électrode (1, 2) sont réalisés sous forme de branches qui s'étendent dans le sens longitudinal de l'instrument et qui sont couplées à une poignée de l'instrument ou sont formées sur ladite poignée pour effectuer un mouvement de pincette, de pince ou de ciseaux.

4. Instrument médical HF selon la revendication 3, **caractérisé en ce que** le support d'électrodes (4) séparé est muni d'une électrode droite ou d'un ensemble d'électrodes (6) droites, s'étendant dans le sens longitudinal de l'instrument.

5. Instrument médical HF selon la revendication 4, **caractérisé en ce que** le support d'électrodes (4) est monté pivotant au moyen de charnières sur l'extrémité distale de la branche d'électrode (1) concernée.

6. Instrument médical HF selon la revendication 5, **caractérisé en ce qu'**un mécanisme de support, de préférence mobile, est disposé sur l'extrémité proximale du support d'électrodes (4) séparé, entre le support d'électrodes (4) et la branche d'électrode (1) concernée, de telle sorte qu'il est possible de régler l'angle de pivotement actif entre le support d'électrodes (4) et la branche d'électrode (1).

7. Instrument médical HF selon l'une quelconque des revendications précédentes, **caractérisé en ce qu'**entre le support d'électrodes (4) et l'électrode ou l'ensemble d'électrodes (6), montés sur ledit support, est insérée une pluralité d'éléments à ressort (30), de préférence des ressorts hélicoïdaux, des ressorts à lames ou des éléments élastomères, par lesquels l'électrode ou l'ensemble d'électrodes (6) sont amenés à distance du support d'électrodes (4) en direction de l'électrode ou de l'ensemble d'électrodes (7) en face.

8. Instrument médical HF selon la revendication 7, **caractérisé en ce que** les éléments à ressort (30) sont respectivement regroupés par paires, lesdites paires d'éléments à ressort étant écartées uniformément les unes des autres dans le sens longitudinal des électrodes.

9. Instrument médical HF selon la revendication 7 ou 8, **caractérisé en ce que** les éléments à ressort (30), en particulier du type ressort hélicoïdal, sont insérés dans des logements qui sont réalisés dans le support d'électrodes (4).

10. Instrument médical HF selon l'une quelconque des revendications précédentes, **caractérisé en ce que** l'électrode ou l'ensemble d'électrodes (6), montés sur le support d'électrodes (4) séparé, sont réalisés en forme de U sur une vue en élévation, une fente dans le sens de la largeur du support d'électrodes (4) se formant entre les branches parallèles des électrodes.

11. Instrument médical HF selon la revendication 10, **caractérisé en ce que** le porte-électrode (1), ainsi que le support d'électrodes (4) séparé, monté sur celui-ci, ont chacun une fente longitudinale couvrant sensiblement la fente entre les branches d'électrodes, à travers laquelle est guidée de manière isolante une électrode de coupe (24) de préférence en forme de lame, de telle sorte que celle-ci, indépendamment du porte-électrode (1) et du support d'électrodes (4) séparé, monté sur ce dernier, est mobile pour la coupe d'un tissu fixé entre les porte-électrode (1, 2).

12. Instrument médical HF selon l'une quelconque des revendications précédentes, **caractérisé en ce que** sur au moins un des porte-électrode (1, 2), de préférence sur un porte-électrode (1) en forme de branche, muni du support d'électrodes (4) séparé, est prévu un dispositif de réglage de distance (32) actionnable manuellement, qui permet de régler et/ou de modifier une distance minimum entre les électrodes.

13. Instrument médical HF selon l'une quelconque des revendications 7 à 9, **caractérisé en ce que** les éléments à ressort (30) sont réalisés en même temps sous forme d'éléments thermoconducteurs, qui sont couplés thermiquement à l'électrode ou l'ensemble d'électrodes (6, 7), ainsi étayés, et au support d'électrodes.
